(19) Europäisches Patentamt
European Patent Office
Office européen des brevets

(11) **EP 3 372 158 B1**

(12) **EUROPÄISCHE PATENTSCHRIFT**

(45) Veröffentlichungstag und Bekanntmachung des Hinweises auf die Patenterteilung:
**12.05.2021 Patentblatt 2021/19**

(51) Int Cl.:
***A61B 5/20*** *(2006.01)* ***A61N 1/36*** *(2006.01)*
***A61B 5/391*** *(2021.01)*

(21) Anmeldenummer: **17000375.0**

(22) Anmeldetag: **08.03.2017**

(54) **SYSTEM ZUR INTRAOPERATIVEN ÜBERWACHUNG DER FUNKTIONSFÄHIGKEIT VON NERVEN**

SYSTEM FOR INTRAOPERATIVE MONITORING OF THE FUNCTIONALITY OF NERVES

SYSTÈME DE SURVEILLANCE AU COURS D'UNE OPÉRATION DE LA CAPACITÉ FONCTIONNELLE DES NERFS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**12.09.2018 Patentblatt 2018/37**

(73) Patentinhaber: **inomed Medizintechnik GmbH
79312 Emmendingen (DE)**

(72) Erfinder:
• **Kaiser, Willi
79312 Emmendingen (DE)**
• **Somerlik - Fuchs, Karin
79111 Freiburg (DE)**
• **Wegner, Celine
79106 Freiburg (DE)**
• **Krüger, Thilo
79312 Emmendingen (DE)**

(74) Vertreter: **Börjes-Pestalozza, Henrich et al
Maucher Jenkins
Patent- und Rechtsanwälte
Urachstraße 23
79102 Freiburg im Breisgau (DE)**

(56) Entgegenhaltungen:
EP-B1- 2 589 410        WO-A1-2016/149829
US-A- 3 893 452         US-A1- 2006 020 218
US-A1- 2016 183 819

**Beschreibung**

Gegenstand der Erfindung

Die Erfindung betrifft ein Überwachungssystem (1), eingerichtet für die in Teilen oder (vorzugsweise) vollständig automatisierte intraoperative Überwachung von Nerven während eines chirurgischen Eingriffs, wobei das Überwachungssystem mindestens jeweils eine Stimulationselektrode zum Einbringen in den Bauchraum und Sensoren zur Messung von Signalen aufweist, wobei als Sensoren mindestens ein Sensor zur Messung des Blasendrucks und mindestens ein Sensor zur Messung der Aktivität des Sphincter Ani vorgesehen sind, wobei der mindestens eine Sensor zur Messung der Blasenfunktion zur Messung des Blasendrucks als Drucksensor eingerichtet und über ein Adapterelement ("Druckmessbox") mit dem übrigen System verbunden ist und der oder die Sensoren zur Messung der Aktivität des Sphincter ani ein oder mehrere Elektroden zur EMG-Messung sind. Auch dieses Überwachungssystem beinhaltende Methoden und Verfahren sind Gegenstand der Offenbarung

Hintergrund der Erfindung

Das pelvine Neuromonitoring ist eine junge Methode des intraoperativen Monitoring, welche vor ca. vier Jahren auf den Markt eingeführt wurde. Diese Technologie und Methode erlaubt die Identifikation, Überwachung und Schonung der Nerven des kleinen Beckens bei Eingriffen wie zum Beispiel der Totalen Mesorektalen Exzision zur Entfernung eines Rektumkarzinoms. Die Methode basiert auf der elektrischen Stimulation der gefährdeten Nervenstrukturen mittels einer Handsonde und der elektromyografischen Überwachung (Elektromyogramm = EMG) des internen analen Schließmuskels (IAS) unter gleichzeitiger Blasenmanometrie. Die Grundzüge der Methode wurden bereits in einem deutschen Patent (DE10 2010 019796 B4) und einer europäischen Patentanmeldung (EP2589410 B1) beschrieben. Der Fokus der vorangegangenen Anmeldung liegt vor allem auf der Kombination des IAS-EMG und der Blasenmanometrie und der Filterung des Signals des IAS in einem Bereich unter 30 Hz, sowie der Methode / des Verfahrens und die Verwendung dieser Anordnung.

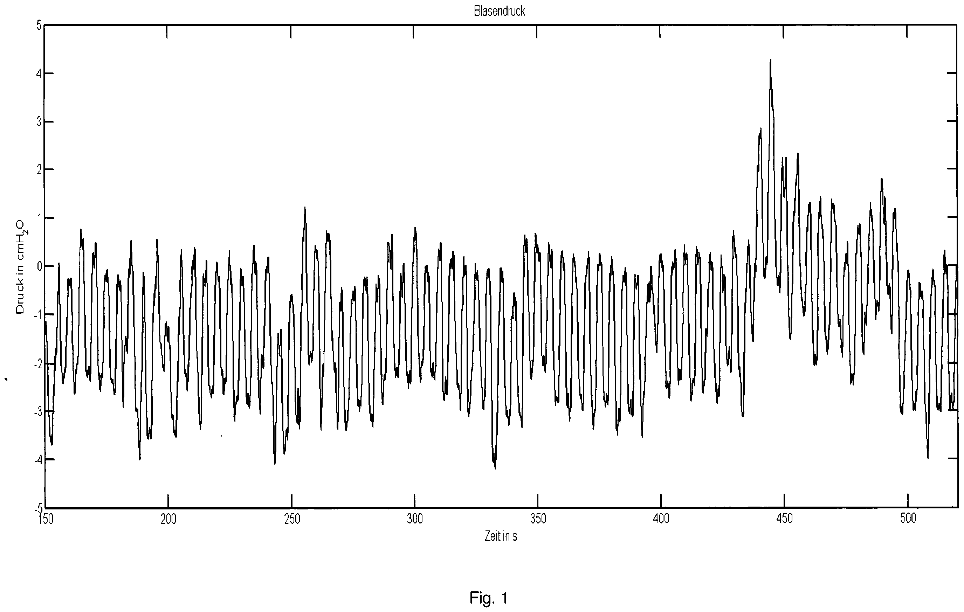

Bei offenen Operationen mit einem großen Bauchschnitt kann das Blasendrucksignal direkt auf dem Bildschirm dargestellt werden. Bei Anstieg des relativen Blasendrucks und Überschreitung eines gewissen Schwellwertes gibt das Neuromonitoring-System ein akustisches und visuelles Feedback an den Operateur, was eine erfolgreiche Stimulation der den Detrusor Vesicae innervierenden Nerven anzeigt. Bei der laparoskopischen Operation hingegen ist der Bauchraum geschlossen und mit einem Gas beaufschlagt, was dem Operateur bessere Sicht verschafft. Dies bedeutet aber, dass ein geschlossenes Drucksystem um die Blase herum aufgebaut wird, wodurch sämtliche Druckänderungen, die auf dieses System einwirken, auf die Blase und auf die Blasenmanometrie übertragen werden und somit das Ergebnis der Druckmessung verfälschen können. In dieser Konstellation ist ein konstanter Einfluss auf die Blasendruckmessung durch die Beatmung des Patienten gegeben. Durch die rhythmischen Bewegungen des Zwerchfells sind im Blasendrucksignal periodische Störungen zu sehen. Diese Störungen können das Signal derart verfälschen, dass kleine Druckänderungen, welche durch eine Detrusorkontraktion hervorgerufen werden, nicht mehr eindeutig auszumachen sind. Fig. 1 zeigt exemplarisch eine Kurve von Messsignalen mitsamt atmungsbedingten Druckänderungen.

Kritisch sind auch der korrekte Aufbau und das korrekte Vornehmen aller Anschlüsse, vor allem des Sensors zur Messung der Blasenfunktion mitsamt dem Adapterelement und der Sensoren zur Messung der Aktivität des Sphincter Ani. Hier sollten Fehlerquellen strikt ausgeschaltet werden und die Anwendbarkeit im Operationssaal derart erleichtert werden, dass Fehler und aufwändiges Betreuen der technischen Seite des Überwachungssystems vermieden werden, um nicht von der eigentlichen Operation und den mittels der Anordnung gewonnenen Informationen abzulenken.

Auch die Messung sexueller Funktionsfähigkeit während einer Operation ist wünschenswert.

Die Aufgabe der Erfindung besteht daher darin, ein Überwachungssystem bereitzustellen, das es ermöglicht, Nerven während laparoskopischer Operationen zu überwachen, eine einfache Benutzerführung aufweist und auch die Messung sexueller Funktionen während einer Operation ermöglicht.

Allgemeine Beschreibung der Erfindung

Erfindungsgemäß wird dies in einer ersten Ausführungsform der Erfindung erreicht durch ein Überwachungssystem der eingangs genannten Art, welches dadurch gekennzeichnet, ist dass das Überwachungssystem für einen laparoskopischen Eingriff ausgestattet ist und

(A) eine softwareimplementierte Unterdrückung von Atemartefakten bei der Messung des Blasendruckes aufweist, wobei das Überwachungssystem zur Unterdrückung von Atemartefakten bei der Messung des Blasendruckes (i) einen Tiefpassfilter in Form eines digitalen Filters beinhaltet, der das bei der Messung des Blasendrucks erhaltene Messsignal glättet, so dass es - mindestens im Wesentlichen - nur das oder die Blasendrucksignale inklusive überlagerter Atmungskurve anzeigt, (ii) eine Abtast- und Halte-Schaltung (Sample und Hold - SUH) beinhaltet, deren größte und kleinste Werte für eine neue Kurve gemittelt und die Mittelwerte bis zum Auftreten neuer solcher größter und kleinster Werte gehalten werden, und dadurch die neue Kurve erhalten-wird, welche als bereinigtes Blasendrucksignal dient.

(B) vorzugsweise weiter eine Benutzerführung aufweist, die die automatische Überprüfung mindestens einer der Bedingungen ausgewählt aus (i) dem korrekten Anschluss des Adapterelements zur Messung des Blasendruckes an das übrige System, (ii) der korrekten Anbringung der Stimulationselektrode(n) und (iii) der korrekten Platzierung der Elektrode(n) zur EMG-Messung im Überwachungssystem beinhaltet,

und (C) in einer weiteren bevorzugten Ausführungsform zusätzlich eine automatisierte Teilinterpretation oder Vollinterpretation der eingehenden Messsignale aufweist, die eine Aufarbeitung und Darstellung der abgeleiteten Messsignale dergestalt beinhaltet, dass der Anwender die für ihn essentielle Information übersichtlich erfassen kann.

Die Offenbarung betrifft auch die Verwendung bzw. ein Verfahren unter Verwendung eines derartigen erfindungsgemäßen Überwachungssystems während einer Operation zur intraoperativen Kontrolle der Funktionsfähigkeit (und damit Unversehrtheit) des Nervensystems bzw. von Nerven im Beckenbereich und/oder unmittelbar angrenzenden Regionen des Bauchraums eines Patienten bei minimal-invasiv chirurgischen Eingriffen z.B. an dem Verdauungsapparat, den Sexualorganen, zur Tumorentfernung und/oder bei korrektiven Eingriffen (jeweils) in der Allgemeinchirurgie, Koloproktologie, Gynäkologie, Urologie, Orthopädie und Neurochirurgie, wobei ein oder mehrere Stimulationselektroden, ein oder mehrere Sensoren für die Messung der Blasenfunktion und ein oder mehrere Sensoren zur Messung der Aktivität des Sphincter Ani Internus und gewünschtenfalls des Sphincter Ani Externus im und am Patienten eingesetzt werden und geprüft wird, ob die Stimulation zu Messsignalen führt, die zeigen, dass die Nervenverbindung zum entsprechenden Erfolgsorgan intakt oder ganz oder teilweise geschädigt ist.

Detaillierte Beschreibung und bevorzugte Ausführungsformen der Erfindung:

Soweit in der vorliegenden Offenbarung allgemeine Begriffe verwendet werden, können ein, mehrere oder alle davon bei einer bestimmten Ausführungsform der Erfindung durch vor- und nachstehend genannte spezifischere Begriffe ersetzt werden, was zu bevorzugten Ausführungsformen der Erfindung führt.

Eine bevorzugte Ausführungsform der Erfindung betrifft ein erfindungsgemäßes Überwachungssystem bzw. dessen Verwendung, welches alle für die erste Ausführungsform der Erfindung oben genannten Merkmale (A), (B) und (C) aufweist.

In allen Ausführungsformen der Erfindung ist das Überwachungssystem dadurch gekennzeichnet, dass es zur Unterdrückung von Atemartefakten bei der Messung des Blasendruckes

(i) einen Tiefpassfilter in Form eines digitalen Filters beinhaltet, z.B. einen Mittelwert-FIR Filter, der das bei der Messung des Blasendrucks erhaltene Messsignal glättet, so dass es - mindestens im Wesentlichen - nur das Blasendrucksignale (resultierend aus der Stimulation) mit überlagerter Atmungskurve anzeigt,

(ii) eine Abtast- und Halte-Schaltung (Sample und Hold - SUH) beinhaltet, durch die die größten und kleinsten Werte gemittelt und bis zum Auftreten neuer solcher Werte (oder bis zu einem Timeout (Pause von beispielsweise 4 bis 10, z.B. 6 Sekunden)) gehalten werden, sodass eine neue Kurve erhalten wird, welche als bereinigtes Messsignal für den Blasendruck (bereinigtes Blasendrucksignal) dient. Vorzugsweise werden nur die bereinigten Blasendrucksignale ohne überlagerte Atmungskurve angezeigt,

wobei die Amplitude des so erhaltenen Blasendrucksignals zur Darstellung z.B. auf ein Balkensymbol oder ein vergleichbares Symbol übertragen wird, und bei Überschreitung des Blasendrucks über einen gewissen Schwellwert (z.B. 1 cmH$_2$O) dem Operateur ein akustisches und visuelles Feedback über die erfolgreiche Stimulation der den Detrusor Vesicae innervierenden Nerven gegeben wird.

Vorteilhaft ist insbesondere in allen Ausführungsformen der Erfindung das Überwachungssystem derart eingerichtet, dass es weiter (iii) ein automatisches softwaregesteuertes Grundlinienkorrekturelement beinhaltet, um weitere im Bauchraum oder in der Blase auftretende Schwankungen der Grundlinie (Nulllinie) der Blasendrucksignale zu berücksichtigen, wobei das Grundlinienkorrekturelement eine Schwankung der Grundlinie - und damit einen Drift (Wandern) des Nullwertes auf den Wert eines letzten ermittelten Minimums - nur dann annimmt und für die weitere Auswertung berücksichtigt, wenn

(a) das detektierte Minimum der Mittelungskurve der Blasendrucksignals aus dem vorstehenden Absatz um einen Betrag von einem vorherigen als Grundlinie - oder Nullwert - gewerteten Minimum abweicht, für den plausibel ist, dass er nicht Ergebnis einer Stimulation mittels einer Stimulationselektrode sein kann, beispielsweise um 0,5 cmH$_2$O oder weniger, z.B. von 0,0 bis 0,5 cmH$_2$O;

und/oder

(b), falls es um einen höheren Betrag abweicht, das detektierte Minimum zu einem Zeitpunkt nach dem letzten detektierten Maximum der Mittelungskurve gemessen wird, für den plausibel ist, dass das Minimum nicht von einer Stimulation mittels einer Stimulationselektrode beeinflusst sein kann, beispielsweise nach mehr als 15 Sekunden; oder

(c) falls keine dieser beiden Bedingungen erfüllt ist, keine Schwankung der Grundlinie annimmt, sondern das Vorliegen einer stimulationsabhängigen Blasendruckänderung, und damit die alte Grundlinie als Nulllinie für die weitere Auswertung des Blasendruckes beibehält.

Durch die Eliminierung der Schwankungen wird die Grundlinie minimal Null und kann keine negativen Werte annehmen, wie es der Fall bei nichtkorrigierten und gestörten Signalen ist. Damit ist eine bessere Erkennung eigentlicher Messwerte (insbesondere der durch Stimulation verursachter Blasendrucksignale) möglich.

In einer weiteren bevorzugten Ausführungsform weist ein Überwachungssystem wie vor- und nachstehend definiert, als Benutzerführung (B) eine solche in softwareimplementierter Ausführungsform auf, die die automatische Überprüfung mindestens einer der Bedingungen ausgewählt aus (i) dem korrekten Anschluss des Adapterelements zur Messung des Blasendruckes an das übrige System, insbesondere mittels Prüfung der Spannungsversorgung des Adapterelementes, (ii) der korrekten Anbringung der Stimulationselektrode(n), insbesondere mittels Kontrolle des applizierten Stromes, und (iii) der korrekten Platzierung der Elektrode(n) zur EMG-Messung, insbesondere mittels. Impedanzmessung, beinhaltet, wobei es vorzugsweise mindestens die unter (i) und (iii) genannten Merkmale aufweist.

Besonders vorteilhaft ist ein Überwachungssystem in allen Ausführungsformen der Erfindung derart eingerichtet, dass es im Rahmen der unter B) genannten Benutzerführung die Spannungsversorgung des Adapterelements zur Messung der Blasenfunktion durch ein softwaregesteuertes Prüfelement überprüft, das prüft, ob eine Trägerfrequenz, welche für die Messung des Blasendruckes verwendet wird, auf dem Messsignal vorhanden ist, und dass die Amplitude der Trägerfrequenz gemessen wird, um zu prüfen, ob der Sensor für den Blasendruck angeschlossen und der Stromkreis geschlossen ist.

Der verwendete Sensor für den Blasendruck kann z.B. ein piezoresistiver Drucksensor sein. Das beinhaltet, dass auf einer Membran im Sensor mehrere Widerstände, z.B vier Widerstände entsprechend der Wheatstone Brücke, angeordnet sind. Bei Druckänderungen im Sensorgehäuse wird die Membran verformt. Dabei werden die Widerstände gestaucht oder gedehnt und ändern dadurch ihre elektrische Eigenschaft, d.h. ihren Widerstand. Diese Schaltung wird mit einer Spannung versorgt. Zum Beispiel kann hierfür ein Trägersignal z.B. als Wechselspannung mit einer Frequenz von 100 bis 10000 Hz (Trägerfrequenz, vorzugsweise als Sinusschwingung), z.B.1000 Hz, einer festgelegten Amplitude verwendet werden (z.B. 0,1 bis 20V, wie z.B. 2V, von Spitze-zu-Spitze). Dieses Signal wird im Adapterelement erzeugt und zu dem Drucksensor geschickt. Durch eine Änderung der Eigenschaften der Widerstände wird im Sensor die Amplitude des Trägersignals verändert. Diese Änderungen in der Amplitude zeigen eine Druckänderung im Messsystem und somit in der Blase an. Das Messsignal des Drucksensors wird über das Adapterelement (Druckmessbox) direkt an den Verstärker einer Steuerungseinheit (umfassend_Datenerfassungs-, Datenauswertungs-, Steuerungs- und Stimulationsmodul, kann jedoch auch das Adapterelement selbst beinhalten) des Überwachungssystems (z.B. C2 NerveMonitor, inomed Medizintechnik GmbH, Emmendingen, Deutschland) und an die Software weitergegeben, wo dieses Trägersignal demoduliert und in Druck in $cmH_2O$ umgerechnet wird. Damit das Adapterelement das Trägersignal erzeugen kann, muss es, z.B. über den USB-Anschluss des Überwachungssystems, mit Spannung versorgt werden. Die Prüfung ob der Sensor für den Blasendruck angeschlossen und der Stromkreis geschlossen ist, erfolgt hier also dadurch, dass das eingehende Signal von dem Adapterelement sowohl auf das Trägersignal mit seiner Frequenz (z.B. 1000 Hz) als auch auf die Amplitude des Signals überprüft wird, und so kann festgestellt werden, ob der USB-Anschluss hergestellt und der Sensor verbunden wurde.

Weitere bevorzugte Ausführungsformen der Erfindung beinhalten ein erfindungsgemäßes Überwachungssystem insbesondere nach einem der drei letzten Absätze, dadurch gekennzeichnet, dass die Überprüfung der Platzierung der Elektrode(n) zur EMG-Messung über ein implementiertes softwaregesteuertes Impedanzmessungselement erfolgt.

Vorteilhaft wird für den Anwender eine Ampeldarstellung der Elektroden gezeigt: Rot bedeutet eine schlecht oder nicht angeschlossene Elektrode, Grün bedeutet eine technisch korrekt platzierte Elektrode. So kann das Ergebnis automatisiert in akzeptabel oder nicht akzeptabel eingestuft werden. Bei nicht akzeptablem Elektrode-Gewebekontakt wird der Nutzer aufgefordert, die Elektroden zu replatzieren. So wird zum Beispiel ein Impedanzwert kleiner als 10 Kilo-Ohm als guter Elektrode-Gewebekontakt akzeptiert und dem Anwender dieses durch die grüne Anzeige bestätigt. Bei einer Impedanz z.B. größer als 10 Kilo-Ohm zeigt die rote Anzeige dem Anwender, dass die Elektrode neu platziert werden muss.

Eine weitere vorteilhafte Erfindungsverkörperung bezieht sich auf Ausführungsformen der Erfindung, die ein Überwachungssystem wie vor- und nachstehend beschrieben beinhalten, wobei der oder die Sensoren zur Messung der Blasenfunktion zur Messung des hydrostatischen Druckes in der Blase ausgelegt sind und mit einem Blasenkatheter verbunden sind oder teilweise oder vollständig integriert sind.

Eine andere bevorzugte Erfindungsverkörperung bezieht sich auf Ausführungsformen der Erfindung, die ein Überwachungssystem wie vor- und nachstehend beschrieben beinhalten, wobei der oder die Sensoren zur Messung der Aktivität des Sphincter Ani Internus als wenigstens eine oder mehrere bipolare EMG-Ableitelektroden in Form von Nadel- oder Oberflächenelektroden ausgestaltet ist oder sind.

Noch eine weitere bevorzugte Erfindungsverkörperung bezieht sich auf Ausführungsformen der Erfindung, die ein Überwachungssystem wie vor- und nachstehend beschrieben beinhalten, welches weiter zur Ableitung der Aktivität des Sphincter Ani Externus eingerichtet ist, wobei der oder die Sensoren zur Messung der Aktivität des Sphincter Ani Externus als wenigstens eine oder mehrere bipolare EMG-Ableitelektroden in Form von Nadel- oder Oberflächenelektroden ausgestaltet ist oder sind.

Die Sensoren und die Stimulationselektrode(n) sind über die Druckmessbox oder direkt mit einer Steuerungseinheit (umfassend Datenerfassungs-, Datenauswertungs-, Steuerungs- und Stimulationsmodul) verbunden, welche vorzugsweise weiter eine Anzeigevorrichtung und optionale beispielsweise computer- und softwaregestützten Auswertungs- und Dokumentationsausstattungen beinhaltet und auch eingerichtet ist zur Aufarbeitung der Messsignale und außerdem zur Beaufschlagung der Stimulationselektrode(n) mit Stimulationspulsen.

Alternativ kann das Adapterelement für die EMG-Messsignale und für den Blasendruck auch durch eine separate Druckmessbox zur Überwachung und Weiterleitung der blasenspezifischen Messdaten und durch eine separate Elektroden-Minibox zur Überwachung und Weiterleitung der EMG-Messsignale ersetzt sein.

Vorteilhaft weist ein Überwachungssystem in Ausführungsformen der Erfindung als mindestens eine Stimulationselektrode für das Hervorrufen von Muskelreaktionen eine handführbare Stimulationssonde und/oder eine Sonde für kontinuierliche Stimulation, oder eine Kombination davon, auf.

Ein erfindungsgemäßes Überwachungssystem ist vorzugsweise eingerichtet zur bzw. wird verwendet bei der intraoperativen Kontrolle der Funktionsfähigkeit des Nervensystems bzw. von Nerven Bauchbereich (insbesondere Beckenbereich und/oder unmittelbar angrenzende Regionen des Bauchraums) eines Patienten bei minimal-invasiv chirurgischen Eingriffen z.B. an dem Verdauungsapparat, den Sexualorganen, zur Tumorentfernung und bei korrektive Eingriffen in der Allgemeinchirurgie, Koloproktologie, Gynäkologie, Urologie, Orthopädie und Neurochirurgie, wobei ein oder mehrere Stimulationselektroden, ein oder mehrere Sensoren für die Messung der Blasenfunktion und ein oder mehrere Sensoren zur Messung der Aktivität des Sphincter Ani Internus und gewünschtenfalls des Sphincter Ani Externus beinhaltet sind und eingesetzt werden und geprüft wird, ob die Stimulation zu Messsignalen führt, die zeigen, ob die Nervenverbindung zum entsprechenden Erfolgsorgan intakt oder ganz oder teilweise geschädigt ist.

Das autonome Nervensystem des Beckenbodens z.B. beinhaltet den Plexus Hypogastricus Superior, der Harnblase, Enddarm und Geschlechtsorgane als Bestandteil des Sympathicus versorgt, hypogastrische Nerven (Nn Hypogastrici), welche den Beckenbodenbereich von diesem ausgehend versorgen, die Nervi Splanchnici Lumbales (Pelvic Splanchnic Nerves, PSN), welche ebenfalls die Blase, Enddarm und Geschlechtsorgane versorgen, und den Plexus Hypogastricus Inferior (PHI), der auch Organe im Beckenraum versorgt, sowie den Pudendus Nerv.

Im vorliegenden Fall werden durch das erfindungsgemäße Überwachungssystem insbesondere die Überwachung des Sphincter Ani Internus (glatter Teil des analen Schließmuskels) und des Detrusor Vesicae realisiert.

Der Sphincter Ani Internus ist ein glatter Muskel, dessen Reaktionsmuster auf eine erfolgreiche Nervenstimulation sich deutlich von dem bekannten Muster der Skelettmuskulatur unterscheidet. Daher ist vorzugsweise eine Anpassung der Messvorrichtung dergestalt vorgesehen, dass diese die Ableitung des EMG der glatten Muskeln, insbesondere des Sphincter Ani Internus, ermöglicht, was am besten bei einer Messung in dem Frequenzbereich zwischen ungefähr 2 und ungefähr 40 Hz erfolgt, zum Beispiel 2 bis 30 Hz, insbesondere 5 bis 25 Hz so dass in einer vorteilhaften Ausgestaltung des erfindungsgemäßen Überwachungssystems und bei dessen Anwendungen ein entsprechender software- und/oder hardware-implementierter Bandpassfilter vorgesehen ist.

Was den Detrusor Vesicae angeht, ist die erfindungsgemäße Kontrollanordnung vorzugsweise zur Messung des Druckes vorgesehen, da der Druck innerhalb der Blase ansteigt, wenn der Muskel kontrahiert. Vorteilhaft ist der Druckmesser als Kombination derart ausgeführt, dass der Blasendruck wie ein EMG aufgenommen werden kann, beispielsweise in Form einer Kombination aus einem Drucksensor und vorteilhaft einer dafür eingerichteten separaten Druckmessbox für eine erste Signalnormierung und -weiterleitung, welche auch die Aufnahme der Signale der Sensoren für die Messung des Sphincter Ani Internus und des Sphincter Ani Externus weiterleiten kann.

Das erfindungsgemäße Überwachungssystem weist vorzugsweise die mindestens eine Stimulationselektrode in Form mindestens einer Sonde für die kontinuierliche (insbesondere elektrische) Stimulation und/oder in Form mindestens einer handführbaren Stimulationssonde **wie in** EP 2 589 410 B1 beschrieben für eine intermittierende (elektrische) Stimulation, oder diese beiden Sondentypen auf. Denkbar ist auch eine Kombination,- welche einen Handgriff zum Platzieren einer (beispielsweise zusammengefalteten) Sonde für kontinuierliche Stimulation beinhaltet, die zum Auffinden geeigneter Orte für die Einbringung der Stimulationsreize, für die intermittierende (während kurzer Abschnitte der Operation) Prüfung während der Operation auf Reizweiterleitung durch die genannten Nerven oder für die kontinuierliche Prüfung geeignet ist, wobei die Sonde für kontinuierliche (während längerer Abschnitte der Operation erfolgende) Stimulation lösbar mit einem Handgriff mit oder ohne Arbeitsteil verbunden ist und dieser Handgriff nach Platzierung der Sonde für die kontinuierliche Stimulation abgenommen werden kann, so dass nur diese Sonde verbleibt.

Für eine kontinuierliche Prüfung während der Operation, ob Stimulationsreize noch über die Beckennerven an den Sphincter Ani Internus und die Blase, insbesondere den Detrusor Vesicae, weitergeleitet werden, kann eine Sonde für kontinuierliche Stimulation wie ebenfalls in EP 2 589 410 B1 beschrieben eingesetzt werden.

Außerdem weist das Überwachungssystem mindestens einen Drucksensor zur Messung der Blasenfunktion auf, der den Druck entweder des Schließmuskels der Blase oder vorzugsweise den hydrostatischen Druck im Inneren der Blase zu messen ausgelegt ist. Hier können sämtliche übliche Drucksensoren, beispielsweise piezoelektrische Sensoren, vorgesehen sein. Vorteilhaft ist eine Kombination aus einem Drucksensor, gekoppelt an einen Blasenkatheter oder zur direkten Anordnung innerhalb der Blase.

Schließlich weist das Überwachungssystem mindestens einen Sensor zur Messung der Aktivität der Sphincter, worunter vor- und nachstehend insbesondere der Spincter Ani (Schließmuskel am Darmende), vorzugsweise der Sphincter Ani Externus oder insbesondere der Sphincter Ani Internus, zu verstehen ist, auf, vorzugsweise in Form einer oder mehrerer, beispielsweise mindestens einer bipolaren, EMG-Ableitelektrode(n), insbesondere wie als bipolare, ggf. mit einem Handgriff ausgestattete EMG-Ableitelektrode in der europäischen Patentschrift EP 2 589 410 B1 beschrieben.

In einer besonderen Ausführungsform ist ein erfindungsgemäßes Überwachungssystem weiter (D) eingerichtet mit einer Messsonde zur Messung und Überprüfung der Intaktheit der Nerven, die für die Sexualfunktionen verantwortlich sind, insbesondere durch Ermittlung der Änderung der Durchblutung, insbesondere von Klitoris oder Penis, z.B. mittels eines Elementes zur Photoplethysmographie oder für Laser Doppler Imaging oder Penisplethysmographie oder Umfangsmessung.

Eine entsprechende die Sexualfunktionen betreffende Messsonde kann derart ausgeführt sein, dass sie z.B. bei der Frau von der Geometrie ähnlich einem Tampon, beim Mann ähnlich einem Band um den Penis entweder mit eingearbeiteten LEDs oder anderen Elementen, oder als Druck- oder Dehnmessstreifen, ausgeführt ist.

Die Auswertung der mit der Messsonde erhaltenen Signale beinhaltet die Messung des Anstiegs der lokalen Aktivität hervorgerufen durch die Stimulation der Beckennerven, entweder mittels Umfangsbestimmung oder mittels Durchblutungsmessung.

Vorteilhaft ist das erfindungsgemäße Überwachungssystem derart ausgestattet, dass die Stimulationssignale, welche beispielsweise über das Kabel für die Stimulation zugeleitet werden können, und die EMG-Signale aufeinander abgestimmt sind, um eine gegenseitige Störung zu verhindern. Gleichzeitig liegt (im Gegensatz zu quergestreifter Muskulatur, wo bereits mit beispielsweise 3 Hz eine Stimulation möglich ist) die Frequenz für die Stimulation des glatten Muskels (wie des Sphincter Ani Internus oder des Detrusor Vesicae) bei ungefähr 20 Hz oder höher bis 100 Hz und liegt vorzugsweise über 20 bis 40 Hz. Das (beispielsweise mit der bipolaren EMG-Ableitelektrode ableitbare) EMG-Signal des glatten Muskels bei Stimulation wird daher vorzugsweise die Aufnahme des Messsignals auf einen Bereich unter 35 Hz, vorzugsweise von 25 Hz oder niedriger, durch entsprechende Filterung mittels eines Bandpassfilters, beispielsweise in dem Adapterelement oder der Steuerungseinheit, beispielsweise zwischen ungefähr 0,5 Hz (vorzugsweise ungefähr 5 Hz) bis ungefähr 25 Hz, gefiltert, denn die Hauptfrequenzkomponenten des glatten Muskels liegen im Bereich unter ungefähr 35 Hz, insbesondere bei 25 Hz oder niedriger.

Umgekehrt kann, sofern auch das EMG des quergestreiften Muskels aufgenommen werden soll, eine andere geeignete Filterung und Stimulationsfrequenz vorgesehen sein. Die entsprechenden Messzyklen für glatten und quergestreiften Muskel können in geeigneter Reihenfolge abwechselnd oder parallel vorgesehen sein.

Beispielsweise kann die Verstärkung für das EMG-Signal des Sphincter Ani Internus 100 bis 5000-fach sein, beispielsweise ungefähr 2500-fach, und die Grenzfrequenz bei 0,01 Hz oder 5 Hz für den Hochpassfilter eingestellt werden, während die Filtereinstellungen für den Sphincter Ani Externus auch eine Verstärkung im angegebenen Bereich und eine Grenzfrequenz von 30 Hz für den Hochpassfilter aufweisen können.

So ist eine genaue zeitliche Korrelation zwischen Stimulation, EMG-Antwortsignalen und Blasendruck durch gleichzeitige Signalweiterleitung möglich.

Für die Stimulation werden beispielsweise Ströme zwischen 1 mA und 15 mA, z.B. zwischen 6 mA oder 12 mA, gewählt. Die Stimulation kann bei 20 bis 40 Hz, beispielsweise bei ungefähr 30 Hz, mit beispielsweise rechteckigen einphasigen Pulsen mit einer Pulsdauer von ungefähr 200 μs erfolgen. Bei der intermittierenden Stimulation sind beispielsweise Pulszüge von 5 bis 30 Sekunden möglich, mit geeigneten Pausen-Intervallen von beispielsweise 5 bis 1.800 Sekunden, z.B. ungefähr 10 bis 120 Sekunden.

"Intraoperativ" bedeutet, dass das erfindungsgemäße Überwachungssystem während einer Operation, insbesondere zur Kontrolle der Integrität von Nerven (vor allem des autonomen Nervensystems im Beckenbereich) verwendet werden kann, also entsprechend ausgestattet ist, das heißt, die Komponenten sind insbesondere nicht zum postoperativen Verbleib im Patienten ausgelegt, somit handelt es sich also nicht um (für längere Zeit postoperativ im Patienten verbleibende) Implantate, und die Stimulationselektroden sind insbesondere auch nicht zur Einleitung (bei fehlender Kontrolle über die Entleerung) oder Unterbindung (im Falle von Inkontinenz) von Darm- und Blasenentleerung eingerichtet und/oder einzubringen. Die Einführung der Überwachungssysteme ist insbesondere selbst nicht Ziel der Operation, sondern sie dienen zur Kontrolle der Nervenintegrität bei Operationen mit anderen Zielen, wie beispielsweise in vor- und nachstehend erwähnten Fachgebieten und darin durchgeführten Operationen. So kann die Vorrichtung insbesondere verwendet werden, es zu vermeiden, Nervenbahnen zu verletzen, was dazu führen kann, dass die Innervierung der für die Kontrolle des Stuhlgangs und der Blasenentleerung und die Sexualfunktionen erforderlichen Muskeln und Gefäße unterbrochen wird, so dass es nicht zu anorektalen, urologischen und sexuellen Dysfunktionen kommt, oder, falls doch Nervenbahnen verletzt werden, zum intraoperativen Vornehmen von Maßnahmen zur Verminderung der negativen Folgen oder zur Vorbereitung geeigneter weiterer derartiger postoperativer Maßnahmen.

Figurenbeschreibung:

Fig. 1: Kurve von Messsignalen des Blasendruckes (x-Achse: Zeit; y-Achse: Spannung) mit überlagerten Atmungsartefakten.

Fig. 2 zeigt ein Ablaufdiagramm für die Unterdrückung von Störungen und Atmungsartefakten.

Fig. 3 zeigt oben die Kurve mit Atmungsartefakten an, unten eine Kurve als Ergebnis einer FIR-Filterung (x-Achse: Zeit; y-Achse: Spannung).

Fig. 4 zeigt graphisch das Ergebnis des SuH-Algorithmus: oben ist das gefilterte Drucksignal analog Fig. 3 unten, unten die Kurve, die aus Mittelung der positiven Werte und negativen Werte und Halten dieser bis zum Auftreten neuer solcher Werte (SuH-Algorithmus) erhalten wird (x-Achse: Zeit; y-Achse: Spannung).

Fig. 5 zeigt exemplarisch ein Flussdiagramm für die automatisierte Überprüfung der Anschlüsse von Adapterelement, Stimulationselektroden und Platzierung der Elektroden zur EMG-Messung in Form einer Benutzerführung.

Fig. 6 zeigt exemplarisch einen Schwarzweiß-Screenshot (in Wirklichkeit erfolgt die vorzugsweise Anzeige farbig) bei Kontrolle der Platzierung von Nadelelektroden oder Rektalelektrode samt Impedanzmessung zur Kontrolle des Gewebekontaktes.

Fig. 7 zeigt exemplarisch einen Schwarzweiß-Screenshot (in Wirklichkeit erfolgt die vorzugsweise Anzeige farbig) bei Kontrolle des Katheteranschlusses für Blasendruckmessung inklusive Kontrolle des korrekten Anschlusses des Sensors und seiner Stromversorgung.

Fig. 8 zeigt exemplarisch einen Schwarzweiß-Screenshot (in Wirklichkeit erfolgt die vorzugsweise Anzeige farbig) im Messmodus eines erfindungsgemäßen Überwachungssystems mit Balkendarstellung.

Fig. 9 zeigt exemplarisch einen Schwarzweiß-Screenshot (in Wirklichkeit erfolgt die vorzugsweise Anzeige farbig) einer Vorschau eines Messreports.

Fig. 10 zeigt exemplarisch einen Schwarzweiß-Screenshot der Signale bei korrektem (unten) und falschem (oben und Mitte) Anschluss des Adapterelements und des Sensors für die Blasendruckmessung.

Fig. 11 zeigt exemplarisch in eine Zustandsmaschine für ein erfindungsgemäßes Überwachungssystem.

Die nachfolgenden Beispiele dienen der Illustration der Erfindung, ohne ihren Umfang einzuschränken.

Beispiele

Beispiel 1: Signalverarbeitung Blasendruck

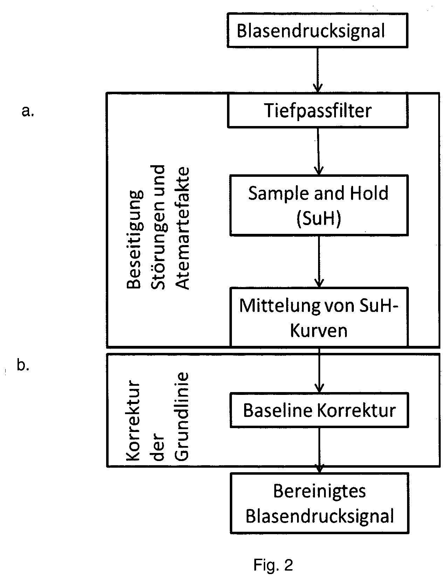

Ein exemplarischer Signalverarbeitungsalgorithmus für eine softwareimplementierte Unterdrückung von Atemartefakten bei der Messung des Blasendruckes ist in Fig. 2 dargestellt.

Dieser Algorithmus kann in zwei Blöcke aufgeteilt werden: Die Filterung der Störungen und Atemartefakte (a.) und die optionale Korrektur der Grundlinie (b.).

a. Das Eingangssignal für den Algorithmus ist das Drucksignal, hier beispielhaft in der Einheit $cmH_2O$ mit einer Abtastfrequenz von 200 Hz. In einem ersten Schritt wird das Signal mit einem FIR Filter der folgenden Form geglättet:

$$y[i] = (x[i] + x[i-1] + \cdots + x[i-(N-1)])/N$$

y: Messpunkt des Ausgangssignals nach dem Filter (Ergebnis der Formel)

x: Ist das Eingangssignal für den Filter

i: Ist die aktuelle Position, also der Samplepunkt, der gerade ausgerechnet wird

N: ist die Länge des Filters

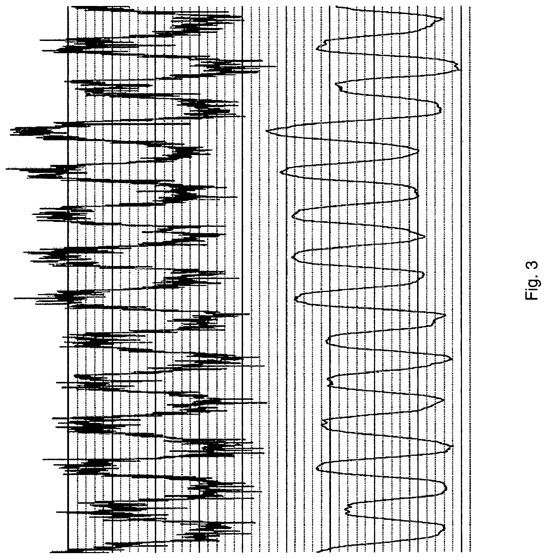

Das Zeitfenster des Filters beträgt hier eine Sekunde (N = 200). Der Effekt des Filters kann in Fig. 3 gesehen werden. Die obere Kurve gibt die Spannungssignale (y-Achse) in Abhängigkeit von der Zeit (x-Achse) mit Atemartefakten an. Die untere (glattere) Kurve zeigt die FIR-gefilterte Form des Signals.

Der nächste Schritt des Algorithmus ist das sogenannte *Sample und Hold* (SuH). Hierbei werden die größten und kleinsten Werte gemittelt und bis zum Auftreten neuer solcher Werte oder bis zu einem Timeout von beispielsweise 6 Sekunden gehalten.

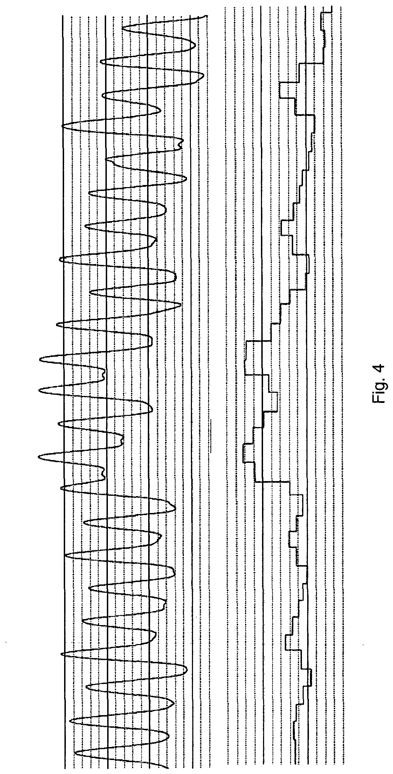

Fig. 4 zeigt oben das durch den FIR-Filter geglättete Signal und unten die ermittelte SuH-Kurve.

Diese Kurve kann entweder direkt auf einem Display als Zeitsignal angezeigt oder in eine Balken- oder unter Zuhilfenahme von Grenzwerten in eine Ampeldarstellung umgewandelt werden. Damit eignet sich die Darstellung direkt für die Bestimmung der durch die Stimulation hervorgerufenen Höhe der Blasendruckänderung.

Da es im Verlauf der Messungen durch übergeordnete Druckänderungen im Bauchraum oder in der Blase zu Schwankungen der Grundlinie im Blasendruck kommen kann (sich füllende Blase, Änderung des Druckes im gasgefüllten Bauchraum), kann es vorteilhaft sein in einem weiteren Schritt eine automatische Korrektur der Grundlinie (b.) zu realisieren.

Ohne den Umfang der Erfindung einzuschränken, kann dies beispielsweise wie folgt erreicht werden: Grundsätzlich wird ein detektiertes Minimum der Kurve als Baseline (Grundlinie) gesetzt und stellt somit die Nullreferenz des Blasendrucks dar. Wenn jedoch ein neues Minimum der durch Mittelung der SuH-Kurven erhaltenen Kurve um mindestens 0,5 cm H2O größer ist als die aktuelle Baseline und innerhalb von 15 Sekunden oder weniger nach dem letzten Maximum auftritt, wird dieses Minimum nicht als neue Baseline akzeptiert und die alte Baseline bleibt bestehen. Die genannten Zahlenwerte: 0,5 cm$H_2$O, 15 Sekunden können jeweils variiert werden (z.B. um das 0,1- bis 10-fache, z.B. das 0,5 bis 2-fache).

Auf diese Weise kann die Baseline automatisch an übergeordnete Druckänderungen angepasst und deren Einfluss somit herausgefiltert werden.

Beispiel 2: Automatisierte Benutzerführung ("Wizard")

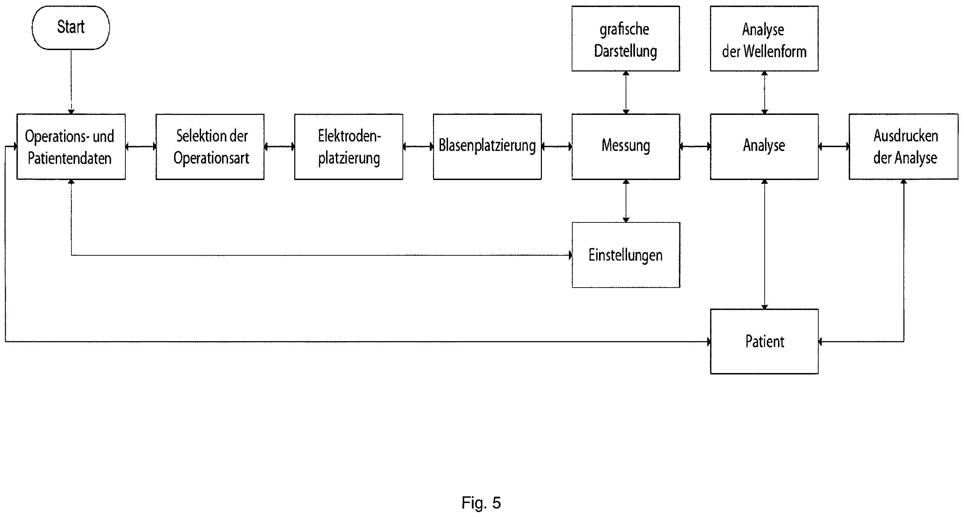

Eine Benutzerführung, die die automatische Überprüfung mindestens einer der Bedingungen ausgewählt aus (i) dem korrekten Anschluss des Adapterelements zur Messung des Blasendruckes an das übrige System, (ii) der korrekten Anbringung der Stimulationselektrode(n) und (iii) der korrekten Platzierung der Elektrode(n) zur EMG-Messung im Überwachungssystem beinhaltet, ist exemplarisch als Flussdiagramm in Fig. 5 dargelegt.

Die Software basiert auf der Standard Anwendung des pelvinen intraoperativen Monitoring wie in EP 2589410 B1 beschrieben: Durch Stimulation mit einer Handsonde werden die autonomen Nerven des kleinen Beckens erregt. Die Funktion der Nerven wird durch die Ableitung verschiedener Muskeln überprüft: Vom Sphincter Ani Internus (glatter Muskel) wird ein EMG-Signal mit einer bipolaren Nadelelektrode oder mit einer Rektalelektrode mit Oberflächenelektrodenkontakten abgeleitet und entsprechend dem Frequenzbereich, in dem die Reaktion erwartet wird (5-25 Hz) gefiltert und aufbereitet. Der Detrusor Vesicae wird indirekt über Messung des Blasendrucks überwacht (siehe oben). Über die EMG-Ableitung des Sphincter Ani Externus als quergestreifter Muskel kann zusätzlich die Relaxation des Patienten kontrolliert werden, damit ein mögliches Übersprechen der Skelettmuskulatur über das Signal des Sphincter Ani Internus detektiert werden kann. Die Darstellung der Signale des Sphincter Ani Internus und der Blasendruckmessung erfolgt über eine vereinfachte Balkenanzeige. Aktionspotentiale des Sphincter Ani Externus, die auf eine ungenügende Relaxation hindeuten, werden automatisch detektiert und ggf. eine entsprechende Warnung angezeigt.

Die Software ist in mehrere Module unterteilt, die den Anwender bei der Verwendung der Methode im OP leiten und unterstützen. Diese Abschnitte sind in Fig. 5 dargestellt und gliedern sich wie folgt. Exemplarische Screenshots der einzelnen Schritte folgen ebenfalls als Figuren.

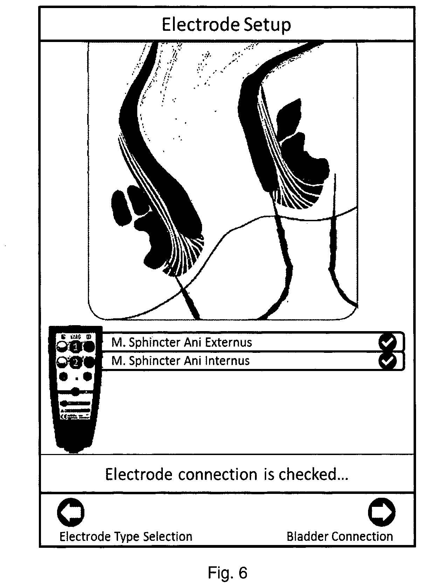

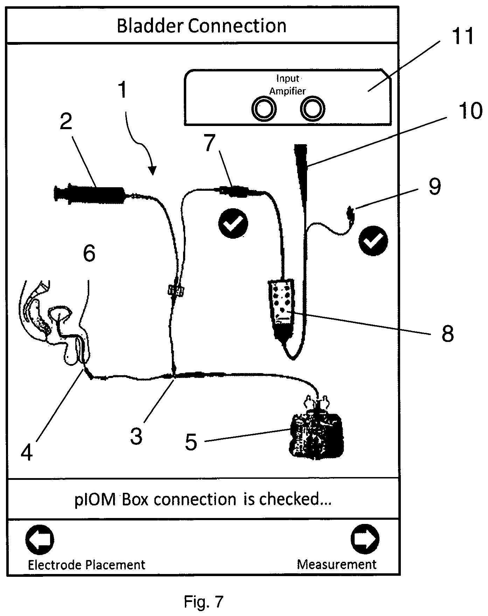

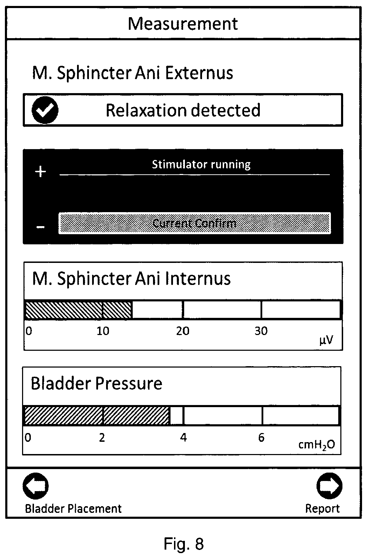

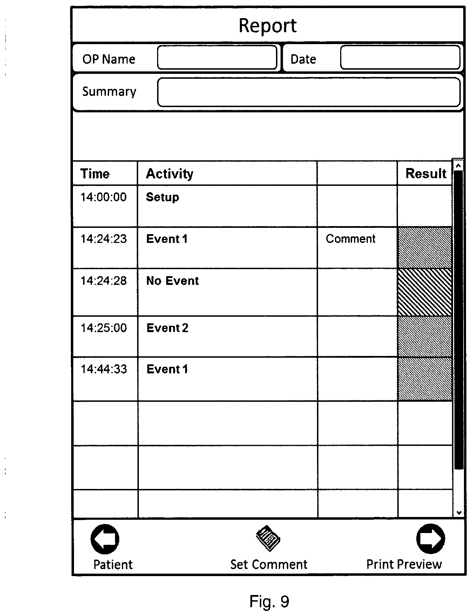

- Eingabe der Patientendaten
- Auswahl des verwendeten Zubehörs für die Ableitung des Sphincter Ani Internus (Nadel- oder Rektalelektrode)
- Platzierung der Nadelelektroden oder der Rektalelektrode inkl. Impedanzmessung zur Kontrolle des Gewebekontaktes (Fig. 6)
- Anschluss des Katheteranschlusssets zur Blasendruckmessung inkl. technischer Kontrolle der korrekten Konnektierung des Sensors und des Adapterelements (Fig. 7)
- Messmodus mit Balkendarstellung (Fig. 8)
- Review und Report mit Druckvorschau des Reports (Fig. 9)

Electrode Setup bedeutet dabei Elektrodeneinrichtung, Electrode connection is checked bedeutet "die Elektrodenverbindung wird geprüft", Electrode Type Selection bedeutet "Auswahl des Elektrodentyps", Bladder Connection bedeutet "Blasenanschluss", Input Amplifier bedeutet "Eingang Verstärker", pIOM Box connection is checked bedeutet "pIOM Box-Anschluss-Verbindung wird geprüft", Electrode Placement bedeutet "Elektrodenplatzierung", Measurement bedeutet "Messung", Relaxation detected bedeutet "Relaxation ermittelt", Stimulator running bedeutet "Stimulator ist aktiv", Current Confirm bedeutet "Strombestätigung", Bladder Pressure bedeutet "Blasendruck", Date bedeutet "Datum", Summary bedeutet "Zusammenfassung", Activity bedeutet "Vorgang", Result bedeutet "Resultat", Setup bedeutet "Einrichtung", Event bedeutet "Ereignis", No Event bedeutet "Kein Ereignis", Comment bedeutet "Kommentar", Set Comment bedeutet "Kommentar festlegen", Print Preview bedeutet "Druckvorschau; die übrigen Ausdrücke haben im Deutschen wie im Englischen die gleiche Schreibweise und Bedeutung.

Bei Fig. 7 bedeuten:

1 Set zur Blasendruckmessung
2 Vorratsgefäße für Saline, die über den Dreiwegehahn 3 mit dem Blasenkathether 4 verbunden sind. Ein Urinbeutel 5 ermöglicht über den Dreiwegehahn 3 die Aufnahme von Urin. Der Drucksensor 6 (beispielsweise ein piezoelektrisches Element) ist über den Konnektor 7 mit der Druckmessbox 8 (die im Gebrauch zugleich auch mit den EMG-Sensorelektroden verbunden ist) verbunden. Die Stromversorgung erfolgt hier über einen USB-Anschluss 9, während der EMG-Anschlusselement 10 mit dem Verstärker 11 als Bestandteil einer Steuerungseinheit (nicht abgebildet), welche Datenerfassungs-, Datenauswertungs-, Steuerungs- und Stimultionsmodule beinhaltet und so als Geber für Stromimpulse, zur Aufnahme der Sensorsignale, zur Auswertung der Signale und zur Steuerung der Vorgänge durch Soft- und Hardware eingerichtet ist.

Die technische Kontrolle des Anschlusses des Drucksensors wird beispielsweise wie folgt durchgeführt:
Im ersten Schritt wird überprüft, ob die Trägerfrequenz auf dem Signal vorhanden ist, was anzeigt, ob die Spannungsversorgung, hier über USB, gewährleistet ist. Der korrekte Anschluss des Adapterelements an den Verstärker - ist im Schritt zuvor bereits Voraussetzung dafür, dass die Elektrodenimpedanz an den Sphincter Ani überhaupt messbar ist.

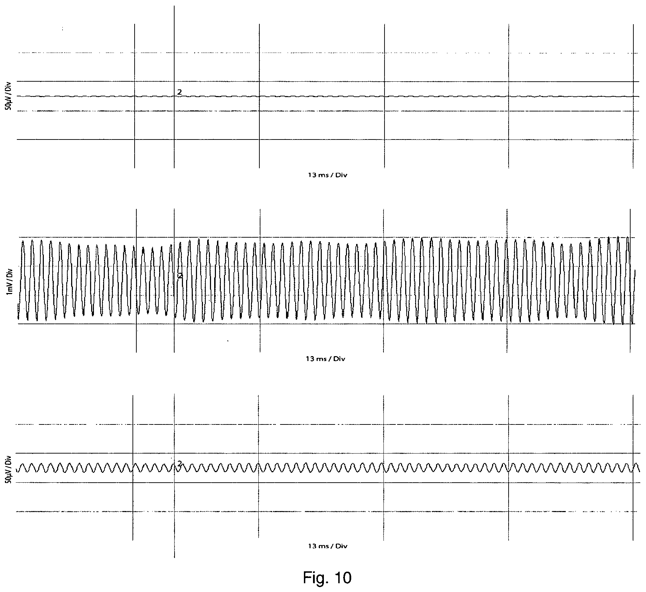

Im zweiten Schritt wird über die Amplitude der Trägerfrequenz kontrolliert, dass der Drucksensor an das Adapterelement angeschlossen und der Stromkreis somit geschlossen ist: Ist er nicht angeschlossen so sind die Kontakte offen, was einen unendlich hohen Widerstand bedeutet. Durch das Übersprechen des Trägersignals auf die übrigen eng beieinanderliegenden Leitungen im Adapterelement resultiert dies in einer großen Amplitude des Trägersignals. In Fig. 10 sind die resultierenden Signale dieser Szenarien dargestellt (Oben: USB-Verbindung ist nicht hergestellt, Mitte: Sensor ist nicht an das Adapterelement angeschlossen, Unten: Korrekter Anschluss sowohl von Adapterelement als auch Sensor).

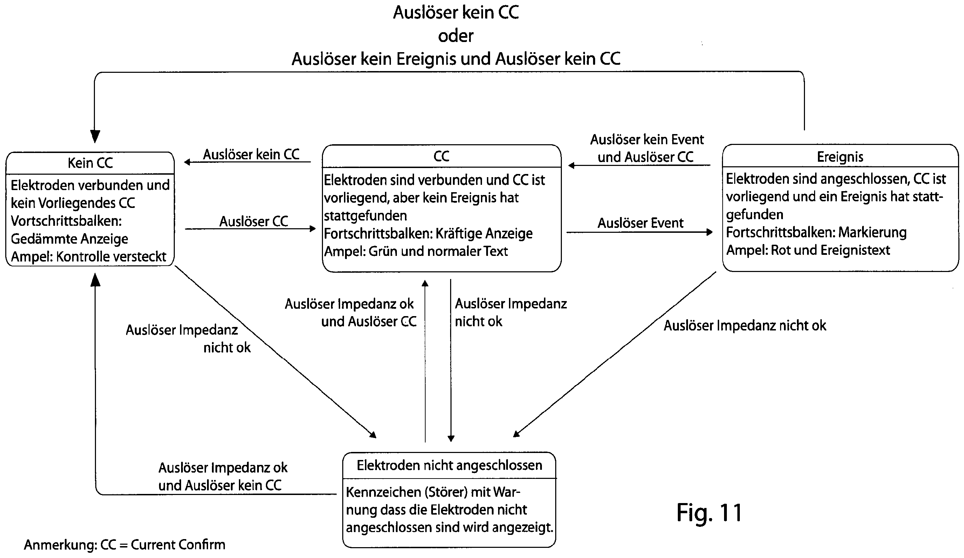

Fig. 11 zeigt exemplarisch eine Darstellung einer Zustandsmaschine (State Machine) für ein erfindungsgemäßes Überwachungssystem.

Verschiedene Zustände des Messmodus der Software im Rahmen einer interoperativen Messung sind dargestellt. Bei detektiertem CC (Current Confirm - also wenn Strom über die Stimulationssonde fließt), wird die Darstellung der Messung über die Balken aktiviert, sofern zuvor eine Initialisierung (Berechnung einer Baseline) über einen Knopfdruck aktiviert und ausgeführt wurde. Bei Überschreitung eines Balkens des Sphincter Ani Internus oder des Blasendrucks über einen gewissen Grenzwert kommt es zu einem sogenannten Ereignis (Event), das sich durch einen für den Balken spezifischen Ton und optisches Feedback (grüne Hinterlegung oder Opakisierung der Anzeige dieses sonst klar dargestellten Balkens) äußert und dem Anwender somit die erfolgreiche Stimulation und die Intaktheit der den Sphincter Ani Internus oder den Detrusor Vesicae innervierenden autonomen Nerven anzeigt. Der Grenzwert für den Blasendruck beträgt zwischen 0,5 und 5 cmH2O, zum Beispiel 1 cmH$_2$O. Der Grenzwert für das Messsignal des Sphincter Ani Internus beträgt zwischen 0,1 und 100 $\mu$V, zum Beispiel zwischen 1 und 10 $\mu$V.

Bei Detektion eines Aktionspotentials in dem Signal des Sphincter Ani Externus wird eine Warnung und ein für dieses Event spezifischer Ton ausgegeben. Der Anwender wird so darüber informiert, dass der Patient nicht vollkommen relaxiert ist und somit ein Übersprechen von Signalen quergestreifter Muskulatur auf das Signal des Sphincter Ani Internus möglich ist.

Für die Stimulation gibt es einen optischen und akustischen Zähler, der den Nutzer anhält, die Stimulationssonde eine gewisse Zeit an derselben Stelle zu halten. Diese Zeit beträgt zwischen 1 und 20 Sekunden, zum Beispiel zwischen 5 und 10 Sekunden, insbesondere 7 Sekunden.

Insbesondere zur sicheren Detektion einer Blasenreaktion ist eine Stimulationsdauer von ungefähr 7 Sekunden wichtig, da der Detrusor Vesicae relativ langsam reagiert und somit der Blasendruckanstieg in der Regel erst nach der o.g. Zeit im Signal zu sehen ist und vom Überwachungssystem detektiert werden kann.

Beispiel 3: Intraoperative Messung

Die intraoperative Messung kann unter Berücksichtigung der obigen Beispiele 1 und 2 analog wie in EP 2 589 410 B1 beschrieben ausgeführt werden. Folgende technische Parameter können beispielsweise intraoperativ verwendet werden:

**Stimulationsmodul**

**Direkte Nervstimulation**

| | |
|---|---|
| Kanäle: | 2 |
| Frequenz: | 1 Hz - 40 Hz frei wählbar |
| Pulsform: | unipolar, negativ, Rechteckpuls |
| Stimulationsstrom: | 0,01 mA - 25 mA frei über Software wählbar |
| Pulsweite: | 200 $\mu$s (50 $\mu$s bis 2000 $\mu$s, frei wählbar) |
| Typ: | Konstantstrom |
| Stimulationsspannung: | max. 100 V |

**Datenerfassungsmodul**

| | |
|---|---|
| Kanäle: | 4/8 |
| Bandbreite: | 0,1 Hz bis 5 kHz |
| Eingangsrauschpegel: | < 1,2 $\mu V_{eff}$ |
| Messbereich: | 800 $\mu V_{pp}$ - 800 m$V_{pp}$ |
| Messgenauigkeit: | +/- 10 % |
| Eingangsimpedanz | 100 M$\Omega$ |
| Auflösung | 16 Bit |
| Abtastrate: | 20 kHz pro Kanal |
| Hochpass Hardware Filter: | 0 Hz (bevorzugt) / 3 Hz / 30 Hz / 120 Hz |
| Tiefpass Hardware Filter: | 5000 Hz / 2500 Hz (bevorzugt)/ 1250 Hz |

Diese Werte sind auch als im allgemeinen Teil der Beschreibung offenbart zu betrachten und mit dortigen Merkmalen kombinierbar für besondere Ausführungsformen der Erfindung.

**Patentansprüche**

**1.** Überwachungssystem (1), eingerichtet für die in Teilen oder vollständig automatisierte intraoperative Überwachung von Nerven während eines chirurgischen Eingriffs, wobei das Überwachungssystem mindestens jeweils eine Stimulationselektrode zum Einbringen in den Bauchraum und Sensoren zur Messung von Signalen aufweist, wobei als Sensoren mindestens ein Sensor zur Messung der Blasenfunktion und mindestens ein Sensor zur Messung der Aktivität des Sphincter Ani vorgesehen sind, wobei der mindestens eine Sensor zur Messung des Blasendrucks als Drucksensor (6) eingerichtet und über ein Adapterelement mit dem übrigen System verbunden ist und der oder die Sensoren zur Messung der Aktivität des Sphincter Ani ein oder mehrere Elektroden zur EMG-Messung sind, **dadurch gekennzeichnet, dass** das Überwachungssystem für einen laparoskopischen Eingriff ausgestattet ist und

(A) eine softwareimplementierte Unterdrückung von Atemartefakten bei der Messung des Blasendruckes aufweist.
wobei das Überwachungssystem (1) zur Unterdrückung von Atemartefakten bei der Messung des Blasendruckes (i) einen Tiefpassfilter in Form eines digitalen Filters beinhaltet, der das bei der Messung des Blasendrucks erhaltene Messsignal glättet, so dass es - mindestens im Wesentlichen - nur das oder die Blasendrucksignale inklusive überlagerter Atmungskurve anzeigt, (ii) eine Abtast- und Halte-Schaltung (Sample und Hold - SUH) beinhaltet, deren größte und kleinste Werte für eine neue Kurve gemittelt und die Mittelwerte bis zum Auftreten neuer solcher größter und kleinster Werte gehalten werden, und dadurch die neue Kurve erhalten-wird, welche als bereinigtes Blasendrucksignal dient.

2. Überwachungssystem nach Anspruch 1, welches

(B) weiter eine Benutzerführung aufweist, die die automatische Überprüfung mindestens einer der Bedingungen ausgewählt aus (i) dem korrekten Anschluss des Adapterelements zur Messung des Blasendruckes an das übrige System, (ii) der korrekten Anbringung der Stimulationselektrode(n) und (iii) der korrekten Platzierung der Elektrode(n) zur EMG-Messung im Überwachungssystem beinhaltet,
und (C) zusätzlich eine automatisierte Teilinterpretation oder Vollinterpretation der eingehenden Messsignale aufweist, die eine Aufarbeitung und Darstellung der abgeleiteten Messsignale dergestalt beinhaltet, dass der Anwender die für ihn essentielle Information übersichtlich erfassen kann.

3. Überwachungssystem nach Anspruch 1, derart eingerichtet, dass es weiter (iii) ein automatisches softwaregesteuertes Grundlinienkorrekturelement beinhaltet, um weitere im Bauchraum oder in der Blase auftretende Schwankungen der Grundlinie (Nulllinie) der Blasendrucksignale zu berücksichtigen, wobei das Grundlinienkorrekturelement eine Schwankung der Grundlinie - und damit einen Drift des Nullwertes auf den Wert eines letzten ermittelten Minimums - nur dann annimmt und für die weitere Auswertung berücksichtigt, wenn (a) das detektierte Minimum der Mittelungskurve des Blasendrucksignals um einen Betrag von einem vorherigen als Grundlinie - oder Nullwert - gewerteten Minimum abweicht, für den plausibel ist, dass er nicht Ergebnis einer Stimulation mittels einer Stimulationselektrode sein kann, beispielsweise um 0,5 cmH$_2$O oder weniger, oder (b), falls es um einen höheren Betrag abweicht, das detektierte Minimum zu einem Zeitpunkt nach dem letzten detektierten Maximum der Mittelungskurve gemessen wird, für den plausibel ist, dass das Minimum nicht von einer Stimulation mittels einer Stimulationselektrode beeinflusst sein kann, beispielsweise nach mehr als 15 Sekunden; oder (c) falls keine dieser beiden Bedingungen erfüllt ist, keine Schwankung der Grundlinie annimmt, sondern das Vorliegen einer stimulationsabhängigen Blasendruckänderung, und damit die alte Grundlinie als Nulllinie für die weitere Auswertung des Blasendruckes beibehält.

4. Überwachungssystem nach einem der Ansprüche 1 bis 3, **dadurch gekennzeichnet, dass** es als Benutzerführung (B) eine solche in softwareimplementierter Ausführungsform aufweist, die die automatische Überprüfung mindestens einer der Bedingungen ausgewählt aus (i) dem korrekten Anschluss des Adapterelements zur Messung des Blasendruckes an das übrige System, insbesondere mittels Prüfung der Spannungsversorgung des Adapterelementes, (ii) der korrekten Anbringung der Stimulationselektrode(n), insbesondere mittels Kontrolle des applizierten Stromes, und (iii) der korrekten Platzierung der Elektrode(n) zur EMG-Messung, insbesondere mittels Impedanzmessung, beinhaltet, wobei es vorzugsweise mindestens die unter (i) und (iii) genannten Merkmale aufweist.

5. Überwachungssystem nach Anspruch 4, **dadurch gekennzeichnet, dass** es derart eingerichtet ist, dass es im Rahmen der unter B) genannten Benutzerführung die Spannungsversorgung des Adapterelements zur Messung der Blasenfunktion durch ein softwaregesteuertes Prüfelement überprüft, das prüft, ob ein Trägersignal mit einer Trägerfrequenz, welche für die Messung des Blasendruckes verwendet wird, auf dem Messsignal vorhanden ist, und die Amplitude des Trägersignals gemessen wird, um zu prüfen, ob der Sensor für den Blasendruck angeschlossen und der Stromkreis geschlossen ist.

6. Überwachungssystem nach einem der Ansprüche 4 oder 5, **dadurch gekennzeichnet, dass** die Überprüfung der Platzierung der Elektrode(n) zur EMG-Messung über ein implementiertes softwaregesteuertes Impedanzmessungselement erfolgt,

7. Überwachungssystem nach einem der Ansprüche 1 bis 6, **dadurch gekennzeichnet, dass** der oder die Sensoren zur Messung der Blasenfunktion zur Messung des hydrostatischen Druckes in der Blase ausgelegt sind und mit einem Blasenkatheter verbunden sind.

8. Überwachungssystem nach einem der Ansprüche 1 bis 7, wobei der oder die Sensoren zur Messung der Aktivität des Sphincter Ani Internus als wenigstens eine oder mehrere bipolare EMG-Ableitelektroden in Form von Nadel- oder Oberflächenelektroden ausgestaltet ist oder sind.

9. Überwachungssystem nach einem der Ansprüche 1 bis 8, **dadurch gekennzeichnet, dass** es weiter zur Ableitung der Aktivität des Sphincter Ani Externus eingerichtet ist.

10. Überwachungssystem nach einem der Ansprüche 1 bis 9, **dadurch gekennzeichnet, dass** die Sensoren und die Stimulationselektrode(n) mit einem Datenerfassungs- und Stimulationsmodul verbunden sind, welches eine Anzeigevorrichtung und optionale beispielsweise computer- und softwaregestützten Auswertungs- und Dokumentationsausstattungen beinhaltet und auch eingerichtet ist zur Aufarbeitung der Messsignale und außerdem zur Beaufschlagung der Stimulationselektrode(n) mit Stimulationspulsen.

11. Überwachungssystem nach einem der Ansprüche 1 bis 10, **dadurch gekennzeichnet, dass** die mindestens eine Stimulationselektrode eine handführbare Stimulationssonde und/oder eine Sonde für kontinuierliche Stimulation, oder eine Kombination davon, darstellt.

12. Überwachungssystem insbesondere nach einem der Ansprüche 1 bis 11, **dadurch gekennzeichnet, dass** es eingerichtet ist zur intraoperativen Kontrolle der Funktionsfähigkeit des Nervensystems bzw. von Nerven im Bauchbereich eines Patienten, insbesondere im Beckenbereich und/oder unmittelbar angrenzenden Regionen des Bauchraums eines Patienten, bei minimal-invasiv chirurgischen Eingriffen z.B. an dem Verdauungsapparat, den Sexualorganen, zur Tumorentfernung und bei korrektiven Eingriffen in der Allgemeinchirurgie, Koloproktologie, Gynäkologie, Urologie, Orthopädie und Neurochirurgie, wobei ein oder mehrere Stimulationselektroden, ein oder mehrere Sensoren für die Messung der Blasenfunktion und ein oder mehrere Sensoren zur Messung der Aktivität des Sphincter Ani Internus und gewünschtenfalls des Sphincter Ani Externus beinhaltet sind und eingesetzt werden und geprüft wird, ob die Stimulation zu Messsignalen führt, die zeigen, dass die Nervenverbindung zum entsprechenden Erfolgsorgan intakt oder ganz oder teilweise geschädigt ist.

13. Überwachungssystem nach einem der Ansprüche 1 bis 12, weiter (D) eingerichtet zur Messung und Überprüfung der Intaktheit der Nerven verantwortlich für die Sexualfunktionen.

14. Überwachungssystem nach Anspruch 13, **dadurch gekennzeichnet, dass** die Messung und Überprüfung der Intaktheit der Nerven durch Ermittlung der Änderung der Durchblutung erfolgt, die verantwortlich sind für die Sexualfunktionen.

15. Überwachungssystem nach Anspruch 14, wobei die Ermittlung der Änderung der Durchblutung mittels eines Elementes zur Photoplethysmographie oder für Laser Doppler Imaging oder Penisplethysmographie oder durch Umfangsmessung erfolgt.

**Claims**

1. Monitoring system (1) set up for the partially or completely automated intraoperative monitoring of nerves during a surgical procedure, wherein the monitoring system has at least one stimulation electrode for introduction into the abdominal space and sensors for the measurement of signals, wherein at least one sensor for measuring the bladder function and at least one sensor for measuring the activity of the anal sphincter are provided as sensors, wherein the at least one sensor for measuring the bladder pressure is set up as a pressure sensor (6) and is connected to the rest of the system via an adapter element, and the one or more sensors for measuring the activity of the anal sphincter are one or more electrodes for EMG measurement, **characterized in that** the monitoring system is equipped for a laparoscopic procedure and

(A) has a software-implemented suppression of respiratory artefacts in the measurement of the bladder pressure, wherein the monitoring system (1) includes, for the suppression of respiratory artefacts in the measurement of the bladder pressure, (i) a lowpass filter in the form of a digital filter, which smooths the measurement signal obtained in the measurement of the bladder pressure, such that the measurement signal indicates, at least in the main, only the one or more bladder pressure signals including the superimposed respiration curve, and (ii) a sample and hold circuit whose greatest and smallest values are averaged for a new curve, and the average values are held until the occurrence of new such greatest and smallest values, thereby obtaining the new curve which serves as a corrected bladder pressure signal.

2. Monitoring system according to Claim 1, which

(B) moreover has a user guide which automatically checks at least one of the conditions selected from (i) the correct connection of the adapter element for measurement of the bladder pressure to the rest of the system, (ii) the correct attachment of the stimulation electrode(s), and (iii) the correct placement of the electrode(s) for the EMG measurement in the monitoring system,

and (C) additionally an automated partial interpretation or full interpretation of the incoming measurement signals, which includes processing and presentation of the derived measurement signals in such a way that the user can clearly grasp the information essential to him.

3. Monitoring system according to Claim 1, set up in such a way that it further includes (iii) an automatic software-controlled baseline correction element in order to take account of further baseline (zero line) fluctuations of the bladder pressure signals occurring in the abdominal space or in the bladder, wherein the baseline correction element assumes a fluctuation of the baseline, and therefore a drift of the zero value to the value of a last determined minimum, and takes it into account for the further evaluation, only if (a) the detected minimum of the averaging curve of the bladder pressure signal deviates from a previous minimum evaluated as baseline, or zero value, by an amount for which it is plausible that it cannot be the result of a stimulation by means of a stimulation electrode, for example by 0.5 cmH$_2$O or less, or (b), if it deviates by a higher amount, the detected minimum at a time after the last detected maximum of the averaging curve is measured for which it is plausible that the minimum cannot be influenced by a stimulation by means of a stimulation electrode, for example after more than 15 seconds; or (c), if neither of these two conditions is met, it assumes no fluctuation of the baseline, but instead the presence of a stimulation-dependent bladder pressure change, and thus maintains the old baseline as the zero line for the further evaluation of the bladder pressure.

4. Monitoring system according to one of Claims 1 to 3, **characterized in that** it has, as user guide (B), a software-implemented embodiment thereof which involves the automatic checking of at least one of the conditions selected from (i) the correct connection of the adapter element for measurement of the bladder pressure to the rest of the system, in particular by testing the voltage supply of the adapter element, (ii) the correct attachment of the stimulation electrode(s), in particular by controlling the applied current, and (iii) the correct placement of the electrode (s) for the EMG measurement, in particular by means of impedance measurement, wherein it preferably has at least the features mentioned under (i) and (iii).

5. Monitoring system according to Claim 4, **characterized in that** it is set up in such a way that, in the context of the user guide mentioned under B), it checks the voltage supply of the adapter element, for measurement of the bladder function, by a software-controlled test element which tests whether a carrier signal with a carrier frequency, which is used for the measurement of the bladder pressure, is present on the measurement signal, and the amplitude of the carrier signal is measured in order to test whether the sensor for the bladder pressure is connected and the current circuit closed.

6. Monitoring system according to either of Claims 4 and 5, **characterized in that** the placement of the electrode (s) for the EMG measurement is checked via an implemented software-controlled impedance measurement element.

7. Monitoring system according to one of Claims 1 to 6, **characterized in that** the one or more sensors for measuring the bladder function are designed to measure the hydrostatic pressure in the bladder and are connected to a bladder catheter.

8. Monitoring system according to one of Claims 1 to 7, wherein the one or more sensors for measuring the activity of the sphincter ani internus is or are designed as at least one or more bipolar EMG lead-off electrodes in the form of needle electrodes or surface electrodes.

9. Monitoring system according to one of Claims 1 to 8, **characterized in that** it is further set up to derive the activity of the sphincter ani externus.

10. Monitoring system according to one of Claims 1 to 9, **characterized in that** the sensors and the stimulation electrode(s) are connected to a data acquisition and stimulation module which includes a display device and optional for example computer and software-supported evaluation and documentation equipment and is also set up to process the measurement signals and also to apply stimulation pulses to the stimulation electrode(s).

11. Monitoring system according to one of Claims 1 to 10, **characterized in that** the at least one stimulation electrode is a hand-held stimulation probe and/or a probe for continuous stimulation, or a combination thereof.

12. Monitoring system in particular according to one of Claims 1 to 11, **characterized in that** it is set up for intraoperative monitoring of the functionality of the nervous system or nerves in the abdominal region of a patient, in particular in the pelvic region and/or directly adjoining regions of the abdominal space of a patient, in minimally invasive surgical procedures, e.g. on the digestive tract, the sex organs, for tumour removal, and in corrective procedures in general surgery, coloproctology, gynaecology, urology, orthopaedics and neurosurgery, wherein one or more stimulation electrodes, one or more sensors for measuring the bladder function and one or more sensors for measuring the activity of the sphincter ani internus and, if so desired, the sphincter ani externus are included and used, and it is checked whether the stimulation leads to measurement signals which show that the nerve connection to the corresponding effector organ is intact or wholly or partially damaged.

13. Monitoring system according to one of Claims 1 to 12, further set up (D) for measuring and checking the integrity of the nerves responsible for the sexual functions.

14. Monitoring system according to Claim 13, **characterized in that** the integrity of the nerves responsible for the sexual functions is measured and checked by determining the change in the blood flow.

15. Monitoring system according to Claim 14, wherein the change in the blood flow is determined by means of an element for photoplethysmography or for laser Doppler imaging or penile plethysmography or by circumference measurement.

**Revendications**

1. **Système de surveillance (1)** configuré pour la surveillance au cours d'une opération partiellement ou entièrement automatisée des nerfs pendant une intervention chirurgicale, ce système de surveillance présentant au moins une électrode de stimulation à insérer dans l'abdomen et des capteurs pour mesurer des signaux, au moins un capteur pour mesurer la fonction vésicale et au moins un capteur pour mesurer l'activité du sphincter anal étant prévus comme capteurs, l'au moins un capteur pour mesurer la pression vésical étant configuré comme un capteur de pression (6) et relié avec le reste du système par un élément adaptateur et le ou les capteurs pour mesurer l'activité du sphincter anal étant une ou plusieurs électrodes de mesure EMG,
**caractérisé en ce que** ce système de surveillance est conçu pour une intervention laparoscopique et

   (A) présente une dépression implantée par logiciel d'artéfacts respiratoires lors de la mesure de la pression vésicale,

   le système de surveillance (1), pour la dépression d'artéfacts respiratoires lors de la mesure de la pression vésicale, (i) contenant un filtre passe-bas sous la forme d'un filtre numérique qui lisse le signal de mesure obtenu lors de la mesure de la pression vésicale, de sorte qu'il indique - au moins pour l'essentiel - uniquement le ou les signaux de pression vésicale avec la courbe respiratoire superposée, (ii) contenant un circuit d'échantillonnage et de maintien (Sample and Hold - SAH) dont la moyenne des valeurs maximum et minimum est établie pour établir une nouvelle courbe et dont les valeurs moyennes sont maintenues jusqu'à l'arrivée de nouvelles valeurs maximum et minimum, la nouvelle courbe qui sert de signal de pression vésicale nettoyé étant ainsi obtenue.

2. Système de surveillance selon la revendication 1, lequel

   (B) présente en outre un guide d'utilisation qui contient la vérification automatique d'au moins une des conditions sélectionnées parmi (i) le raccordement correct de l'élément adaptateur pour mesurer la pression vésicale au reste du système, (ii) le montage correct de l'électrode/des électrodes de stimulation et (iii) le placement correct de l'électrode/des électrodes de mesure EMG dans le système de surveillance,
   et (C) présente de plus une interprétation partielle ou une interprétation totale automatisée des signaux de mesure entrants qui contient un traitement et une représentation des signaux de mesure déduits, de façon que l'utilisateur peut saisir clairement l'information essentielle pour lui.

3. Système de surveillance selon la revendication 1, configuré de sorte (iii) qui contient en outre un élément de correction automatique de la ligne base contrôlé par logiciel pour prendre en compte d'autres variations de la ligne de fond (ligne zéro) survenant dans l'abdomen ou dans la vessie, dans lequel le système de correction de ligne de base ne suppose, et ne prend en compte pour la suite de l'évaluation, une variation de la ligne de fond - et donc une dérive de la valeur zéro sur la valeur d'un dernier minimum établi que si

(a) le minimum détecté de la courbe moyenne du signal de pression vésicale s'écarte d'une quantité du minimum précédemment évalué comme ligne de fond - ou valeur zéro - pour laquelle il est plausible qu'elle puisse ne pas être le résultat d'une stimulation au moyen d'une électrode de stimulation, par exemple de 0,5 cm de $H_2O$ ou moins, ou

(b), dans le cas où il s'écarte d'une quantité plus importante, le minimum détecté est mesuré à un moment postérieur au dernier maximum détecté de la courbe de moyenne, pour laquelle il est plausible que le minimum ne peut pas être influencé par une stimulation au moyen d'une électrode de stimulation, par exemple après plus de 15 secondes, ou

(c), dans le cas où aucune de ces deux conditions n'est remplie, il ne supposer aucune variation de la ligne de fond, mais la présence d'une variation de pression vésicale liée à la stimulation, et conserve donc la ligne de fond comme ligne zéro pour la suite de l'évaluation de la pression vésicale.

4. Système de surveillance selon une des revendications 1 à 3, **caractérisé en ce qu'**il présente en guise de guide d'utilisation (B) une forme de réalisation implantée dans le logiciel qui contient la vérification automatique d'au moins une des conditions sélectionnées parmi (i) le raccordement correct de l'élément adaptateur pour mesurer la pression vésicale au reste du système, en particulier au moyen du contrôle de l'alimentation électrique de l'élément adaptateur, (ii) le montage correct de l'électrode/des électrodes de stimulation, en particulier au moyen du contrôle du courant appliqué, et (iii) le placement correct de l'électrode/des électrodes de mesure EMG, en particulier au moyen d'une mesure d'impédance, étant entendu qu'il présente de préférence au moins les caractéristiques mentionnées aux points (i) et (iii).

5. Système de surveillance selon la revendication 4, **caractérisé en ce qu'**il est configuré de sorte qu'il vérifie dans le cadre du guide utilisateur mentionné au point B) l'alimentation électrique de l'élément adaptateur pour mesurer la fonction vésicale à l'aide d'un élément de contrôle contrôlé par logiciel qui contrôle si un signal porteur avec une fréquence porteuse qui est utilisée pour la mesure de la pression vésicale est présent sur le signal de mesure, et l'amplitude du signal porteur est mesuré pour contrôler si le capteur pour la pression vésicale est raccordé et si le circuit électrique est fermé.

6. Système de surveillance selon une des revendications 4 ou 5, **caractérisé en ce que** la vérification du placement de l'électrode/des électrodes de mesure EMG se fait par le biais d'un élément de mesure d'impédance contrôlé par logiciel implanté.

7. Système de surveillance selon une des revendications 1 à 6, **caractérisé en ce que** le ou les capteurs pour mesurer la fonction vésicale sont conçus pour mesurer la pression hydrostatique dans la vessie et sont reliés avec un cathéter vésical.

8. Système de surveillance selon une des revendications 1 à 7, dans lequel le ou les capteurs pour mesurer l'activité du sphincter anal interne est ou sont configurés comme au moins une ou plusieurs électrodes de référence EMG bipolaires sous la forme d'électrodes à aiguille ou de surface.

9. Système de surveillance selon une des revendications 1 à 8, **caractérisé en ce qu'**il est en outre configuré pour déduire l'activité du sphincter anal externe.

10. Système de surveillance selon une des revendications 1 à 9, **caractérisé en ce que** les capteurs et l'électrode/les électrodes de stimulation sont reliés avec un module d'enregistrement de données et de stimulation qui contient un dispositif d'affichage et, par exemple, des équipements d'évaluation et de documentation assistés par ordinateur et par logiciel facultatifs et est aussi configuré pour le traitement des signaux de mesure et également pour l'application d'impulsions de stimulation à l'électrode/aux électrodes de stimulation.

11. Système de surveillance selon une des revendications 1 à 10, **caractérisé en ce que** l'au moins une électrode de stimulation constitue une sonde de stimulation à main et/ou une sonde de stimulation continue ou une combinaison de celles-ci.

12. Système de surveillance selon une des revendications 1 à 11, **caractérisé en ce qu'**il est configuré pour le contrôle en cours d'opération de la fonctionnalité du système nerveux ou des nerfs dans l'abdomen d'un patient, en particulier dans la région pelvienne et/ou dans les régions immédiatement contigües de l'abdomen d'un patient, lors d'interventions chirurgicales mini-invasives, p. ex. au niveau de l'appareil digestif ou des organes sexuels, pour l'élimination de tumeurs et lors d'interventions correctives en chirurgie générale, en coloproctologie, en gynécologie, en urologie, en orthopédie et en neurochirurgie, dans lequel une ou plusieurs électrodes de stimulation, un ou plusieurs capteurs pour la mesure de la fonction vésicale et un ou plusieurs capteurs pour la mesure de l'activité du sphincter anal interne et éventuellement du sphincter anal externe sont contenus et utilisés et il est contrôlé si la stimulation génère des signaux de mesure qui indiquent que la liaison nerveuse avec l'organe correspondant est intacte ou entièrement ou partiellement endommagée.

13. Système de surveillance selon une des revendications 1 à 12, configuré en outre (D) pour mesurer et vérifier l'intégrité des nerfs responsables des fonctions sexuelles.

14. Système de surveillance selon la revendication 13, **caractérisé en ce que** la mesure et la vérification de l'intégrité des nerfs qui sont responsables des fonctions sexuelles se fait en déterminant la modification de la circulation sanguine.

15. Système de surveillance selon la revendication 14, dans lequel la détermination de la modification de la circulation sanguine se fait au moyen d'un outil de photopléthysmographie ou d'imagerie doppler à laser ou de pénispléthysmographie ou par mesure de la circonférence.

Fig. 1

Fig. 2

a. Beseitigung Störungen und Atemartefakte

- Blasendrucksignal
- Tiefpassfilter
- Sample and Hold (SuH)
- Mittelung von SuH-Kurven

b. Korrektur der Grundlinie

- Baseline Korrektur
- Bereinigtes Blasendrucksignal

Fig. 3

Fig. 4

Start → Operations- und Patientendaten → Selektion der Operationsart → Elektrodenplatzierung → Blasenplatzierung → Messung → Analyse → Ausdrucken der Analyse

grafische Darstellung ↔ Messung ↔ Einstellungen

Analyse der Wellenform ↔ Analyse ↔ Patient

Fig. 5

Fig. 6

Fig. 7

EP 3 372 158 B1

## Measurement

### M. Sphincter Ani Externus

✔ Relaxation detected

Stimulator running

\+

−

Current Confirm

### M. Sphincter Ani Internus

| 0 | 10 | 20 | 30 | |
|---|----|----|----|---|
| | | | | µV |

### Bladder Pressure

| 0 | 2 | 4 | 6 | |
|---|---|---|---|---|
| | | | | cmH$_2$O |

← Bladder Placement

→ Report

Fig. 8

## Report

| OP Name | | Date | |
|---|---|---|---|
| Summary | | | |

| Time | Activity | | Result |
|---|---|---|---|
| 14:00:00 | **Setup** | | |
| 14:24:23 | **Event 1** | Comment | |
| 14:24:28 | **No Event** | | |
| 14:25:00 | **Event 2** | | |
| 14:44:33 | **Event 1** | | |
| | | | |
| | | | |
| | | | |

Patient          Set Comment          Print Preview

Fig. 9

Fig. 10

Auslöser kein CC
oder
Auslöser kein Ereignis und Auslöser kein CC

Auslöser kein CC → 

**Kein CC**
Elektroden verbunden und kein Vorliegendes CC
Vortschrittsbalken:
Gedämmte Anzeige
Ampel: Kontrolle versteckt

Auslöser CC →

**CC**
Elektroden sind verbunden und CC ist vorliegend, aber kein Ereignis hat stattgefunden
Fortschrittsbalken: Kräftige Anzeige
Ampel: Grün und normaler Text

Auslöser kein Event und Auslöser CC →

Auslöser Event →

**Ereignis**
Elektroden sind angeschlossen, CC ist vorliegend und ein Ereignis hat stattgefunden
Fortschrittsbalken: Markierung
Ampel: Rot und Ereignistext

Auslöser Impedanz nicht ok

Auslöser Impedanz ok und Auslöser CC

Auslöser Impedanz nicht ok

Auslöser Impedanz nicht ok

**Elektroden nicht angeschlossen**
Kennzeichen (Störer) mit Warnung dass die Elektroden nicht angeschlossen sind wird angezeigt.

Auslöser Impedanz ok und Auslöser kein CC

Anmerkung: CC = Current Confirm

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010019796 B4

- EP 2589410 B1